(19) Europäisches Patentamt
European Patent Office
Office européen des brevets

(11) Publication number: **0 168 197**
**A2**

# (12) EUROPEAN PATENT APPLICATION

(21) Application number: 85304529.2

(22) Date of filing: 25.06.85

(51) Int. Cl.⁴: C 07 D 461/00
A 61 K 31/475

(30) Priority: 29.06.84 JP 132926/84
04.03.85 JP 41235/84

(43) Date of publication of application:
15.01.86 Bulletin 86/3

(84) Designated Contracting States:
CH DE FR GB IT LI

(71) Applicant: SANWA KAGAKU KENKYUSHO CO., LTD.
No. 35, Higashi-sotobori-cho
Higashi-ku Nagoya-shi Aichi-ken(JP)

(72) Inventor: Ban, Masatoshi
5-40, Kano-sakaemachi-dori
Gifu-shi Gifu-ken(JP)

(72) Inventor: Baba, Yutaka
3-81, Daichi-shinmachi
Iwakura-shi Aichi-ken(JP)

(72) Inventor: Sawai, Kiichi
1-36-14, Ninomiya
Funabashi-shi Chiba-ken(JP)

(72) Inventor: Kurono, Masayasu
1-7-17, Kakeage Minami-ku
Nagoya-shi Aichi-ken(JP)

(72) Inventor: Hayashi, Motohide
2345-1410, Higashiyama-cho
Kasugai-shi Aichi-ken(JP)

(74) Representative: Diamond, Bryan Clive et al,
Gee & Co. Chancery House Chancery Lane
London WC2A 1QU(GB)

## (54) Eburnamonine oxime derivatives and their preparation and pharmaceutical compositions containing them.

(57) An eburnamonine oxime is of the formula:

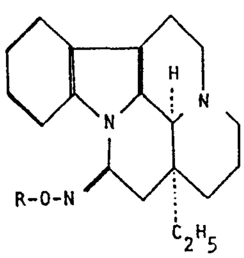

where R is hydrogen, alkyl, alkoxyalkyl, oxisilanyalkyl group or a group

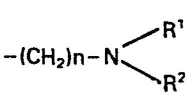 or 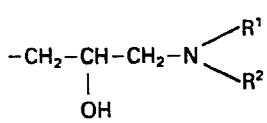

(a) (b)

where $R^1$ and $R^2$ are each hydrogen, C1-4 alkyl, aryl or aralkyl group, or $R^1$ with $R^2$ and the neighbouring N atom completes an optionally substituted heterocyclic ring, and n = 2 or 3.

Preparation is from the thione (R-O-N= replaceby S=) by reaction (i) with hydroxylamine HCL in an organic solvent in the presence of alkali metal carbonate, and (ii) with a halide of R (R not being H or group (b)), if necessary followed by (ii) reaction with Halo-$CH_2CH(OH)_2CH_2$+ + NHR $^1$ $R^2$ to provide group (b), and optionally converting the resultant base to its non-toxic acid addition salt.

A pharmaceutical composition, e.g. injectable solution, tablet or suppository, is useful in improving cerebral blood flow and metabolism, and therefore for treatment of disturbance of brain function, with a superior effect than that of vinpocetine or vincamine derivatives (known alkaloids).

EP 0 168 197 A2

Croydon Printing Company Ltd

# EBURNAMONINE OXIME DERIVATIVES AND THEIR PREPARATION AND PHARMACEUTICAL COMPOSITIONS CONTAINING THEM

The present invention relates to novel eburnamonine oxime derivatives, salts thereof, a process for their manufacture as well as pharmaceutical compositions containing the compound as an effective component. These heterocylic compounds of the invention have good cerebral vasodilating and metabolic activating actions and thus are useful as an effective component of a pharmaceutical agent to cure various disease due to cerebral function disturbances.

It is known that the plant Periwinkle (Vinca minor L. Apocyaceae) contains various alkaloids e.g. vincamine, vincine, vincaminine and vincinine. Among them, vincamine has been employed as an ameliorant for curing cerebral circulatory disturbances. However, vincamine has a disadvantage that its effective action ceases in a relatively short time period.

In order to overcome the disadvantage in at least some measure, various vincamine derivatives, for instance the following, have been proposed.

a)

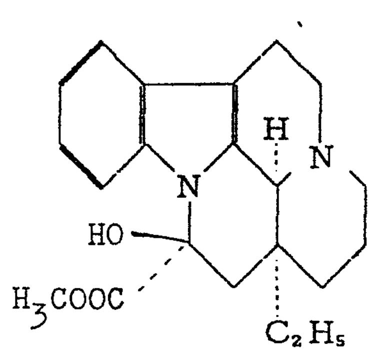

[Pharmacometrics, 10(2), 199-206, 1975]

b)

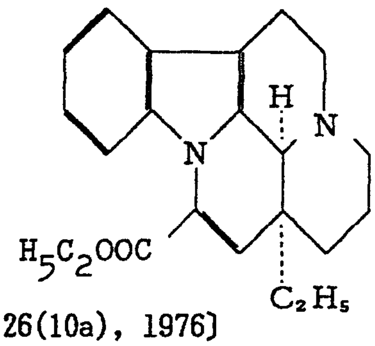

[Arzneim Forsch, 26(10a), 1976]

c)

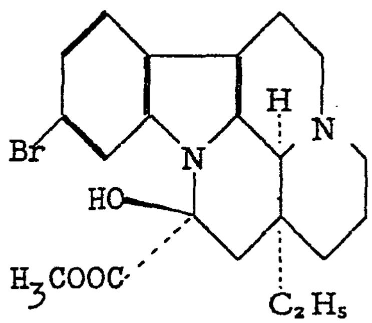

(Belgian Pat. No. 823 409 and British Pat. No. 1 492 579)

d)

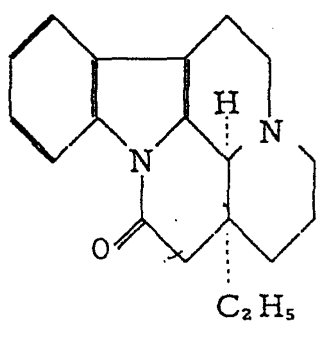

(Arzneim Forsch, 29, 1094, 1979)

However, such known vincamine derivatives have problems in that pharmacological actions thereof are somewhat or remarkably low and the

sustenance of their action is not sufficient.

The object of the invention is to overcome said disadvantages and solve the problems of the vincamine and the synthesized vincamine derivatives, by substituting novel compounds for such known compounds, which have a chemical structure analogous with the latter.

According to one of aspects of the invention, there are provided novel eburnamonine oxime derivatives represented by the following formula (I) as well as salts thereof.

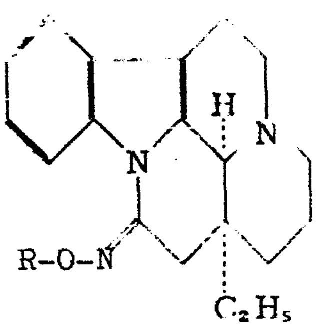

(I)

wherein R is hydrogen, alkyl, alkoxyalkyl or oxisilanylalkyl group or a group of

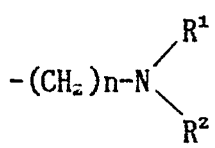

or

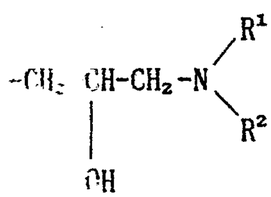

$R^1$ and $R^2$ are the same different and are each hydrogen, alkyl having 1 to 4 carbon atoms, aryl or aralkyl group, or $R^1$ is together with $R^2$ and the neighbouring nitrogen atom an unsubstituted or substituted heterocyclic ring, and n is an integer of 2 or 3.

For the substituent R, examples of "alkyl" are methyl, ethyl, propyl or n-butyl and examples of "alkoxyalkyl"

are methoxymethyl, ethoxymethyl, methoxyethyl, ethoxyethyl or propyloxyethyl and the like.

For the substituents $R^1$ and $R^2$, examples of "alkyl" are methyl, ethyl, propyl or n-butyl, examples of "aryl" are phenyl, o- or p-methoxyphenyl, o- or p-chlorophenyl and benzoyl, of "aralkyl" are benzyl, phenylethyl and benzoylmethyl, of a "non-substituted heterocyclic ring" are pyrrolidinyl, piperidinyl, morpholinyl and piperadinyl, and of a "substituted heterocyclic ring" are a substituted piperadino wherein a substituent therefor may be alkyl such as ethyl, propyl or isopropyl, or aryl such as o- or p-methoxyphenyl or o- or p-chlorophenyl.

According to another aspect of the invention, there is provided a process for the preparation of the eburnamonine oxime derivatives represented by said formula (I).

An eburnamonine oxime of formula (I), wherein R is hydrogen, namely the compound represented by formula (I-a)

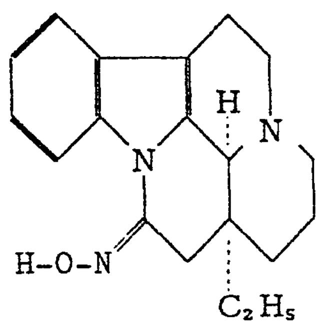

(I-a)

can be prepared by reacting an eburnamoninethione represented by formula (II)

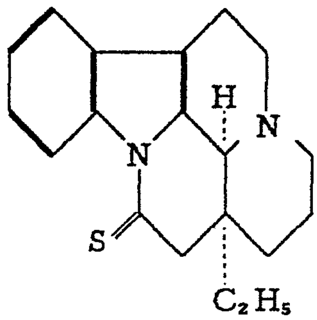

(II)

(this starting material may be obtained according to a conventional method as disclosed by H. Behringer et al in French Pat. No. 2 253 502 and C. A. 84: 90391q) with hydroxylamine hydrochloride in an organic solvent and in the presence of an alkali metal carbonate. In this case, it is preferable that an excess amount of potassium carbonate is employed as the alkali metal carbonate and the reaction is carried out at a temperature of the boiling point of the solvent, for instance ethanol. After completion of the reaction, the solvent is removed under a reduced pressure to concentrate resulting reaction product, and methanol is added thereto to obtain a substance insoluble in methanol through filtration. The substance is washed with water, dried and recrystallized from ethanol to obtain the desired eburnamonine oxime (Compound I-a).

An eburnamonine oxime derivative of Formula I, wherein R is alkyl, alkoxyalkyl, oxisilanylalkyl or a group

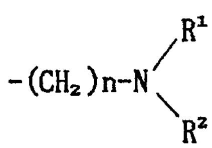

wherein $R^1$, $R^2$ and n have the meanings as referred to, namely the compound represented by formula

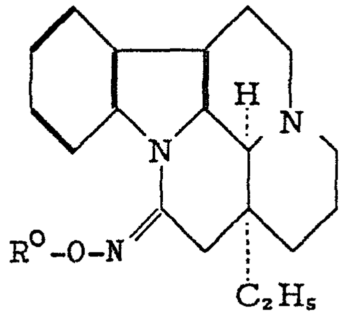

(I-b)

wherein $R^0$ is alkyl, alkoxyalkyl or oxisilanylalkyl group or a group of

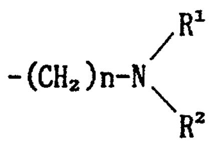

and $R^1$, $R^2$ and n have the meanings referred to, can be prepared by reacting the eburnamonine oxime shown by said formula I-a with an alkali metal hydride in an anhydrous organic solvent and then further reacting the resulting eburnamonine oxime alkali metal salt with a compound represented by formula

$$R^0\text{-}X$$

wherein $R^0$ has the meaning referred to and X is a halogen, in an anhydrous organic solvent.

An eburnamonine oxime derivative of Formula (I), wherein R is the group

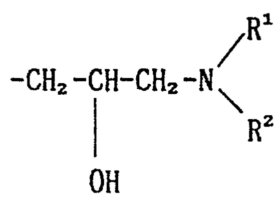

wherein $R^1$ and $R^2$ have the meanings referred to, namely the compound represented by the formula

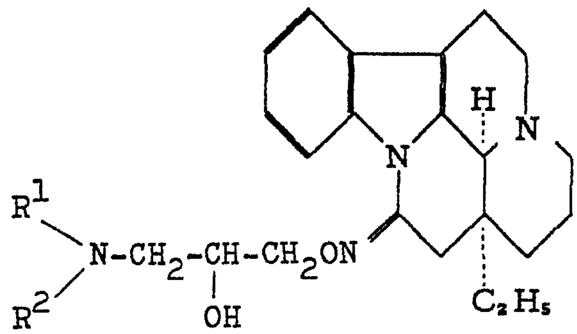
(I-c)

wherein $R^1$, $R^2$ have the meanings referred to, can be prepared by reacting the eburnamonine oxime shown by said formula I-a with an alkali metal hydride in an anhydrous organic solvent, reacting the resulting eburnamonine oxime alkali metal salt with a compound represented by the formula

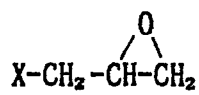

wherein X is a halogen,

in an anhydrous organic solvent, and then further reacting the resulting compound with a compound represented by formula

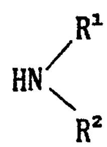

wherein $R^1$ and $R^2$ have the meanings referred to.

As the anhydrous organic solvent, dimethylformamide, dimethylacetamide and dimethylsulfoxide are examples. A temperature between $0^{\circ}$ and $100^{\circ}C$, and more particularly $0^{\circ}$ and $60^{\circ}C$ is preferable for the reaction. Purification of the desired compound can be made by chromatography or any other known method.

Each of the compounds represented by Formula I (Formulae I-a, I-b and I-c) can, if necessary, be converted by a known method to a pharmaceutically acceptable acid addition salt such as hydrochloride, sulfate, phosphate, succinate, maleate, fumarate, citrate, malate, lactate, tartarate, methanesulfonate, benzoate or pamoate.

According to another aspect of the invention, there is provided

a cerebral function improving agent which comprises an eburnamonine-oxime of Formula I or a non-toxic acid addition salt thereof, as an effective component therefor.

By the process of the invention, each of the desired eburnamonine oxime derivatives can be obtained with a relatively high yield and in a relatively easy manner, by starting from an eburnamenine thione obtained according to a conventional process.

The eburnamonine oxime (Formula I-a) and its derivatives (Formulae I-b and I-c) exhibit excellent cerebral vasodilating and metabolic activating actions and thus those are useful as meliorants of cerebral blood circulation and metabolism to cure various diseases due to cerebral circulatory disturbances and/or reduction of oxygen-utilizing function, for instance apoplexy (by meningorrhea, cerebral thrombus or cerebral embolus), cerebral arteriosclerosis, hypertensive cerebral circulatory insufficiency, sequelae due to cephalic injury, retention defect, cephalagia, bradyacusia, tinnitus, diziness, Meniere's disease, retinopathy and the like. At least some of the compounds (I) are superior to the conventional compounds such as vinpocetine and vincamine derivatives in its pharmacological actions and sustenance thereof, which make a reduction of amount of dosage or dosing frequency possible. Further, the toxicity of the compounds (Formula I) is lower than known analogous compounds, so as to increase safety in dosage.

The invention will now be further explained with reference to Examples for preparing Compounds (I), Test Examples showing pharmacological characters of the compounds, and examples of the pharmaceutical compositions.

Example 1

(3α,16α)-eburnamenin-14(15H)-one oxime

A mixture of 2g (8.1 mmol) of (3α,16α)-eburnamenin-14(15H)-thione, 0.62g (9.7 mmol) of hydroxylamine hydrochloride, 3.37g (24.2 mmol) of potassium carbonate and 200 ml of ethanol was refluxed for 4 hours under nitrogen atmosphere and then the solvent was removed under a reduced pressure. To a residue, 100 ml of methanol was added to obtain through filtration a substance insoluble in methanol. The substance was washed with methanol and then water, and dried to obtain 1.78g of desired compound in the form of crude crystals. After recrystallization from ethanol, 1.61g of colorless prisms was obtained.

Free base

Melting point: 285 - 290℃ (dec.)

Yield : 64%

Mass spectrum (m/e): 309 ($M^+$)

NMR spectrum (DMSO-$d^6$) δ ppm:

8.2 - 7.8 (1H, m, Ar-H)

7.3 - 6.8 (3H, m, Ar-H)

3.80 (1H, br s, $C_{3\alpha}$-H)

3.5 - 1.0 (14H, m, $-CH_2$)

0.85 (3H, br t, J=7.0Hz, $-CH_2\underline{CH_3}$)

Hydrochloride

Colorless prisms

Melting point: 288 - 291℃ (dec.) (methanol)

Elementary analysis: $C_{19}H_{23}N_3O \cdot HCl$

Calculated ; C 65.98, H 6.99, N 12.15

Found ; C 66.07, H 6.94, N 12.00

Example 2

(3α,16α)-eburnamenin-14(15H)-one O-[2-(N,N-diethylamino)ethyl]oxime

0.31g (1.0 mmol) of (3α,16α)-eburnamenin-14(15H)-one oxime (Example 1) was suspended under nitrogen gas atmosphere in 5 ml of dimethylformamide and then at 0℃, 55mg (1.5 mmol) of 66% sodium hydride was added thereto to stir the mixture for 2 hours at room temperature (30℃). To the resulting reaction mixture, 0.5 ml of diethylaminoethylchloride was added to stir for further 2 hours at room temperature. After evaporation under a reduced pressure, 5 ml of water was added and extraction was effected with chloroform three times. A combined extract was washed with water, dried with anhydrous sodium sulfate and concentrated under a reduced pressure to obtain as a yellowish oil 0.52g of the desired compound. The crude oil was chromatographed (silica gel, triethylamine:ether:n-hexane = 1:1:10 - 1:4:6) to obtain 0.34g of pale yellow oil.

Free base

Yield: 84%

Mass spectrum (m/e): 408($M^+$)

NMR spectrum ($CDCl_3$) δ ppm:

8.5 - 8.2 (1H, m, Ar-H)
7.6 - 7.0 (3H, m, Ar-H)
4.22 (2H, t, J=6.0Hz, $-OCH_2-$)
3.83 (1H, br s, $C_{3\alpha}$-H)
2.84 (2H, t, J=6.0Hz, $-CH_2-N-$)
3.6 - 1.0 (14H, m, $-CH_2-$)
2.62 (4H, q, J=7.0Hz, $N-\underline{CH_2}CH_3$)
0.95 (6H, t, J=7.0Hz, $N-CH_2\underline{CH_3}$)
0.92 (3H, br t, J=7.0Hz, $-C_2H_5$)

Dihydrochloride-monohydride

Colorless crystals

Melting point: 198 - 201℃ (dec.) (ethanol/ether)

Elementary analysis: $C_{25}H_{36}N_4O \cdot 2HCl \cdot H_2O$

Calculated ; C 60.11, H 8.07, N 11.22

Found ; C 60.17, H 7.90, N 11.11

Example 3

(3α,16α)-eburnamenin-14(15H)-one O-[2-(N,N-dimethylamino)ethyl]oxime

7.00g (22.6 mmol) of (3α,16α)-eburnamenin-14(15H)-one oxime (Example 1) was suspended in 112 ml of dried dimethylformamide. Under argon atmosphere, 2.4g (62.2 mmol) of 60% sodium hydride was added to the suspension while cooling with ice to stir the same for 10 minutes and then the mixture was further stirred for 2 hours at room temperature. The reaction mixture was cooled with ice again, to add 4.89g (33.9 mmol) of N,N-dimethylaminoethylchloride (hydrochloride) and stir for 10 minutes, and then the resulting mixture was further stirred for 2 hours at room temperature. After evaporation, 80 ml of water and 100 ml of dichloromethane were added to separate and obtain an organic phase. The remaining water phase was extracted four times with dichloromethane. The organic phases were combined together, dried with anhydrous sodium sulfate and concentrated under a reduced pressure to obtain 11.0g of the desired compound as a brown oil. The crude oil was chromatographed (silica gel, chroloform : methanol = 40 : 1) to obtain 6.54g of the compound. The compound was converted into its hydrochloride with 34.4% hydrochloric acid/ethanol solution and then the hydrochloride was recrystallized from a mixed solvent of ethanol and ether to obtain 5.50g of dihydrochloride of the desired compound, as colorless needles.

Free base

Yield: quantitative

Mass spectrum (m/e): 380($M^+$)

NMR spectrum ($CDCl_3$) δ ppm:

| | |
|---|---|
| 8.47 - 8.20 | (1H, m, Ar-H) |
| 7.50 - 7.10 | (3H, m, Ar-H) |
| 4.25 | (2H, t, J=6.0Hz, $-CH_2-O-N=$) |
| 3.82 | (1H, brs, $C_{3\alpha}-H$) |
| 3.67 - 0.68 | (18H, m, $-CH_2-$) |
| 2.73 | [2H, t, J=6.0Hz, $-CH_2-N(CH_3)_2$] |
| 2.35 | [6H, s, $-N(CH_3)_2$] |
| 0.92 | (3H, t, J=7.5Hz, $-CH_3$) |

Dihydrochloride

Colorless needles

Melting point: 182 - 194℃ (dec.) (ethanol/ether)

Elementary analysis: $C_{23}H_{32}N_4O\cdot 2HCl\cdot 3/2\ H_2O$

Calculated ; C 57.49, H 7.76, N 11.66

Found ; C 57.38, H 7.74, N 11.67

Example 4

(3α,16α)-eburnamenin-14(15H)-one O-[2-(1-pyrrolidinyl)ethyl]oxime

7.00g (22.6 mmol) of (3α,16α)-eburnamenin-14(15H)-one oxime (Example 1) was suspended in 112 ml of dried dimethylformamide. Under argon atmosphere, 2.49g (62.2 mmol) of 60% sodium hydride was added to the suspension while cooling with ice to stir the same for 10 minutes and then the mixture was further stirred for 2 hours at room temperature. The reaction mixture was cooled with ice again, to add 5.77g (33.9 mmol) of pyrrolidinylethyl chloride (hydrochloride) and stir for 10 minutes, and then the resulting mixture was further stirred for 2 hours at room temperature. After evaporation under a reduced pressure, 60 ml of water and 100 ml of dichloromethane were

added to the residue to dissolve the same and separately obtain an organic phase. The water phase was extracted with dichloromethane three times. The organic phases were combined together, dried with anhydrous sodium sulfate and concentrated under a reduced pressure to obtain 10.9g of the desired compound as a brown oil. The crude oil was chromatographed (silica gel, n-hexane : ether : triethylamine = 6:4:1) to obtain 8.58g of the desired compound as a yellow oil. 7.5g of the yello oil was dissolved in 20 ml of ethanol, 4.60g of 34.4% hydrochloric acid/ethanol solution was added dropwise thereto and then the solvent was removed. Recrystallization was effected from a mixed solvent of ethanol and ether to obtain 6.35g of dihydrochloride of the desired compound, as colorless needles.

<u>Free base</u>

Yield: 94%

Mass spectrum (m/e): 406($M^+$)

NMR spectrum ($CDCl_3$) $\delta$ ppm:

8.52 - 8.13 (1H, m, Ar-H)
7.57 - 7.00 (3H, m, Ar-H)
4.28 (2H, t, J=6.0Hz, $-CH_2-O-N=$)
4.0 - 0.7 (18H, m, $-CH_2-$)
3.85 (1H, brs, $C_{3\alpha}$-H)
2.87 (2H, t, J=6.0Hz, $N-CH_2-$)
2.73 - 2.25 (4H, m, 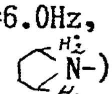)
1.97 - 1.60 (4H, m, 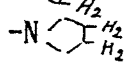)
0.93 (3H, t, J=7.0Hz, $-CH_3$)

<u>Dihydrochloride</u>

Colorless needles

Melting point: 183 - 189℃ (dec.) (ethanol/ether)

Elementary analysis: $C_{25}H_{34}N_4O \cdot 2HCl \cdot H_2O$

Calculated ; C 60.39, H 7.70, N 11.20

Found ; C 60.39, H 7.79, N 11.20

Example 5

(3α,16α)-eburnamenin 14(15H)-one O-{2-[4-(2-methoxyphenyl)-1-piperadinyl]ethyl}oxime

6.48g (21.0 mmol) of (3α,16α)-eburnamenin-14(15H)-one oxime was suspended in 78 ml of dimethylformamide. Under argon atmosphere, 1.47g (36.8 mmol) of 60% sodium hydride were added to the suspension while cooling with ice to stir the same for four hours at room temperature. To the resulting solution, 8.02g (31.5 mmol) of 1-(2-chloroethyl)-4-(2-methoxyphenyl)-1-piperadine in 26 ml of dimethylformamide was added over a time period of 10 minutes, the mixture was stirred for two hours and then the solvent therein was removed therefrom under a reduced pressure.100 ml of water and 200 ml of chloroform were added to extract and obtain an organic phase. The organic phase was dried with anhydrous sodium sulfate and then the solvent was removed under a reduced pressure. The residue was chromatographed (silica gel, ethyl acetate : n-hexane = 1 : 1) to obtain 11.1g of the desired compound as a yellow oil. The oil was further chromatographed (silica gel column, ether : n-hexane : triethylamine = 1 : 1 : 0.05), resulting oil was dissolved in ethanol and converted into hydrochloride with use of hydrochloric acid/ethanol solution. The hydrochloride was recrystallized from a mixed solvent of methanol and ether to obtain 8.71g of same as colorless needles.

Free base

Yield: quantitative

Mass spectrum (m/e): 527 ($M^+$)

NMR spectrum ($CDCl_3$) δ ppm:

8.44 - 8.13 (1H, m, Ar-H)
7.51 - 6.59 (7H, m, Ar-H)
4.31 (2H, t, J=5.5Hz, $-CH_2-O-N=$)
3.81 (3H, s, $-OCH_3$)
3.53 - 0.64 (28H, m, $-CH_2-$ and $-CH_3$)
0.89 (3H, t, J=7.0Hz, $-CH_3$)

Trihydrochloride

Colorless needles

Melting point: 220 - 224℃ (dec.) (methanol/ether)

Elementary analysis: $C_{32}H_{41}N_5O_2 \cdot 3HCl \cdot 1/2\ H_2O$

Calculated ; C 59.48, H 7.02, N 10.84
Found ; C 59.20, H 7.01, N 11.00

Example 6

(3α,16α)-eburnamenin-14(15H)-one O-{2-hydroxy-3-[4-(2-methoxyphenyl)-1-piperadinyl]propyl}oxime

8.00g (25.9 mmol) of (3α,16α)-eburnamenin-14(15H)-one oxime (Example 1) was suspended in dried dimethylformamide. Under argon atomosphere, 1.24g (31.0 mmol) of 60% sodium hydride were added to the suspension while cooling with ice to stir for two hours at room temperature. 4.78g of chloromethyloxirane was added dropwise and the mixture was stirred for 2 hours at room temperature. After evaporation under a reduced pressure, 100 ml of water and 150 ml of dichloromethane were added to the residue to dissolve the same and separately obtain an organic phase. The water phase were extracted twice with dichloromethane. The organic layers were dried and evaporat ed to obtain an oily residue. The residue was chromatographed (silica gel column, chloroform : methanol = 40 : 1) to obtain 9.51g (100%) of (3α,16α)-eburnamenin-14(15H)-one O-(2,3-epoxypropyl)oxime, as a brown viscous oil.

8.00g (21.9 mmol) of the viscous oil and 5.05g (26.3 mmol) of 1-(2-methoxyphenyl)piperadine were dissolved in 100 ml of n-butanol and the solution was refluxed for one hour on an oil bath under argon atmosphere. After evaporation, the residue was purified with column chromatography (silica gel column, chloroform : methanol = 40 : 1) to obtain 11.1g of the desired compound, as a yellow oil.

9.50g of the oil was dissolved in 50 ml of ethanol and 6.00g (56.5 mmol) of 34.4% hydrochloric acid/ethanol solution was added dropwise to convert the free base to its hydrochloride. The solvent in the solution was removed under a reduced pressure and then recrystallization was effected from a mixed solvent of methanol and ether to obtain 7.20g of the hydrochloride of desired compound, as white powder.

Free base

Yield: 91%

Mass spectrum (m/e): 557($M^+$)

NMR spectrum ($CDCl_3$) $\delta$ ppm:

8.50 - 8.20 (1H, m, Ar-H)

7.77 - 6.66 (7H, m, Ar-H)

4.33 - 4.00 (3H, m, $-CH_2-O-N=$, $C_{3\alpha}-H$)

3.82 (3H, s, $-OCH_3$)

4.00 - 0.70 (25H, m, $-CH_2-$, $C_{3\alpha}-H$)

0.99 (3H, t, J=7.0Hz, $-CH_3$)

Trihydrochloride

White powder

Melting point: 171 - 181℃ (dec.) (methanol/ether)

Elementary analysis: $C_{33}H_{43}N_5O_3 \cdot 3HCl \cdot 3/2\ H_2O$

Calculated ; C 57.10, H 7.12, N 10.09

Found ; C 57.00, H 6.95, N 10.07

Pharmacological Test Example 1

(Cerebral Blood Flow Increase)

Male and female mongrel dogs anesthetized with nembutal (sodium pentobarbital) were employed as subject and blood flow in vertebral artery of those animals was measured with use of an electromagnetic rheometer. Each test compound was administered to vertebral artery in a ratio of 1.0 mg/dog to determine change in blood flow in the vertebral artery. Results are shown in following Table 1.

As seen from the Table, the compounds according to the invention have a cerebral blood flow increasing effect which is substantially equivalent to or exceeds that in conventional agent of vinpocetine, as control.

Table 1

| Agent or Compound | Increase in Blood Flow (%) |
|---|---|
| Examples | |
| 1 | 144.9 |
| 2 | 269.5 |
| 3 | 247.3 |
| 4 | 260.6 |
| 5 | 179.3 |
| 6 | 357.7 |
| Vinpocetine | 261.4 |

Pharmacological Test Example 2

(Cerebral Metabolic Activation)

According to a known method [Fujishima et al, "Rinshou-to-Kenkyu (The Clinics and Studies)" 51, 3532, 1974], both common carotid arteries of rats with an essential hypertension (SHR, male, 250 - 350g, 4 to 5 animals/group) were ligated to cause a cerebral ischemia. After one hour from the ligation, each test compound was orally administered in an amount of 10 mg/kg and various parameters in brain

were measured at the time having lapsed 5 hours from the ligation. Results are shown in following Table 2.

From the results, it can be judged that the compounds according to the invention have an improving effect on cerebral energy metabolism, which is substantially equivalent to or exceeds that of vinpocetine as control.

Table 2

| Compound | Amount (μmol/g) | | |
|---|---|---|---|
| | A T P | Lactic acid | Glucose |
| Given no compound | | | |
| no treatment | 0.51 | 5.70 | 368.8 |
| treated | 0.12 | 7.05 | 377.2 |
| Examples | | | |
| 1 | 0.20 | 7.10 | 592.3 |
| 2 | 0.36 | 4.62 | 1028.1 |
| 3 | 0.18 | 7.01 | 740.4 |
| 4 | 0.25 | 6.34 | 895.5 |
| 5 | 0.28 | 5.50 | 802.3 |
| 6 | 0.32 | 4.95 | 1010.5 |
| vinpocetine | 0.36 | 7.00 | 1121.0 |

Pharmacological Test Example 3

(Cerebral Function Protection)

To each of male d,d-mice (16 - 24g, 6 to 24 animals/group), each test. compound was orally administered in an amount of 10 mg/kg. After 30 minutes from the administration, a cylindrical rod made of phenolic resin (diameter: 1cm, weight:36g) was freely dropped from a level height of 40 cm on a vertex of the mouse to hypnotize the same, in accordance with a method disclosed by Manaka et al ["Iyaku-no-Ayumi (The Progress in Medicine )", 104 (4), 253, 1978]. Observations were continued until a recovering reflex from the lethargy and a spontaneous motion appear, to determine a reduction ratio of time period on such behavior appearances. Results are shown in following Table 3.

From the results, it can be judged that the compounds acccording to the invention have a cerebral function protecting effect which is substantially equivalent to or exceeds that of vinpocetine as control.

Table 3

| Compound | Reduction ratio (%) | |
|---|---|---|
| | time to recover sit-up reflex | time to appear spontaneous motion |
| Examples | | |
| 1 | -12.4 | -5.3 |
| 2 | 54.3 | 48.7 |
| 3 | -33.0 | -11.3 |
| 4 | -96.1 | -71.6 |
| 5 | 0.4 | 6.9 |
| 6 | 23.5 | 47.2 |
| vinpocetine | 39.5 | 40.4 |

Pharmacological Test Example 4

(Depression on Blood Platelet Aggregation)

With use of a blood-plasma with excess platelets (obtained from a Wister male rat, about 250g), a depressing effect of each testing compound to ADP and collagen aggregations was studied in vitro (3 to 5 samples/group). Results are shown in following Table 4.

From the results, it can be judged that the compounds according to the invention show a low effect on depression of ADP aggregation but a higher effect on depression of collagen aggregation, which is substantially equivalent or exceeds that of vinpocetine as control.

Table 4

| Compound | Final Conc. ($\mu g/ml$) | depression Ratio (%) to Collagen | depression Ratio (%) to ADP |
|---|---|---|---|
| Examples | | | |
| 1 | 10 | 5.4 | -1.2 |
| 2 | 2 | 73.8 | 5.7 |
| 3 | 10 | 3.2 | 5.0 |
| 4 | 10 | 4.4 | -10.0 |
| 5 | 10 | 9.3 | 5.5 |
| 6 | 10 | 9.5 | 6.3 |
| vinpocetine | 10 | 4.0 | -0.2 |

Pharmacological Test Example 5

(Acceleration on Erythrocyte Deformability)

Each test compound was orally administered to an amount of 10 mg/kg to Wister male rats (about 250g, 5 animals/group). After one hour from the administration, blood sample was collected to prepare 0.1% erythrocyte suspension. An action of the compound to an erythrocyte deformability was studied in accordance with a filtration-pressure method to obtain results as shown in following Table 5.

From the results, it can be judged that the compounds according to the invention have an accelerating action on erythrocyte deformability, although somewhat weaker than that of vinpocetine as control.

Table 5

| Compound | Reduction Rate in Filtering Pressure (%) |
|---|---|
| Examples | |
| 1 | 8.3 |
| 2 | 6.0 |
| 3 | 10.2 |
| 4 | 9.3 |
| 5 | 13.2 |
| 6 | 35.1 |
| vinpocetine | 46.3 |

Pharmacological Test Example 6

(Acute Toxicity)

Each test compound was orally administered to male d,d mice (8 to 10 animals/group) and an $LD_{50}$ value thereon was calculated in accordance with the conventional Litchfield-Wilcoxon method and based on mortality rate at after 72 hours. Results are shown in following Table 6.

From the results, it can be seen that the toxicity of each compound according to the invention is substantially the same as that of vinpocetine as control.

Table 6

| Compound | $LD_{50}$ (mg/kg) |
|---|---|
| Examples | |
| 1 | 510 |
| 2 | 611 |
| 3 | 700 |
| 4 | 532 |
| 5 | 660 |
| 6 | 560 |
| vinpocetine | 796 |

Drug Preparation Example 1 (Tablet)

The following components were added in the specified amounts to prepare tablets in a known manner.

| | |
|---|---|
| Compound (Example 2) | 5 (mg) |
| Crystal cellulose | 40 |
| Magnesium stearate | 2 |
| Hydroxypropylmethylcellulose | 2 |
| Lactate | (suitable amount) |
| Total | 150 mg/tablet |

Drug Preparation Example 2 (Injectable solution)

An isotonic solution for a compound (Example 2) was prepared in a conventional manner and with use of sodium chloride and adjusted its concentration to 2 mg/ml to prepare an injectable solution.

Drug Preparation Example 3 (Suppository)

A compound (Example 6) was added to and uniformly dispersed in cacao fat, in the following ratio and the resulting composition was subjected to a conventional molding process to prepare suppositories.

| | | |
|---|---|---|
| Compound (Example 6) | | 10 (mg) |
| Cacao fat | | 1690 |
| | Total | 1700 mg/suppository |

CLAIMS:

1. An eburnamonine oxime derivative represented by the formula

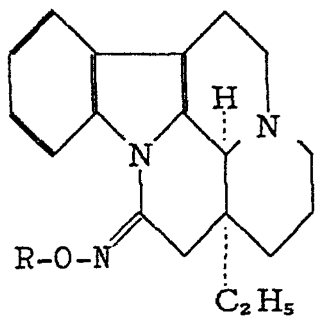

and characterised by the R-O-N group, wherein R is hydrogen, an alkyl, alkoxyalkyl or oxisilanylalkyl group, or a group.

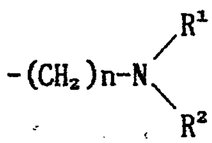
(a)

or

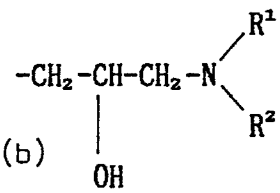
(b)

wherein $R^1$ and $R^2$ are the same or different and are respectively hydrogen, an alkyl having 1 to 4 carbon atoms, aryl or aralkyl group, or $R^1$ represents together with $R^2$ and the neighbouring nitrogen atom an unsubstituted or substituted heterocyclic ring, and n is an integer of 2 or 3,
or a non-toxic salt thereof.

2. (3α,16α)-Eburnamenin-14(15H)-one O-[2-(N,N-dimethylamino)-ethyl]oxime or a non-toxic salt thereof.

3. (3α,16α)-Eburnamenin-14(15H)-one O-[2-(N,N-dimethylamino)-ethyl]oxime or a non-toxic salt thereof.

4. (3α,16α)-Eburnamenin-14(15H)-one O-[2-(1-pyrrolidinyl)-ethyl]oxime or a non-toxic salt thereof.

5. (3α, 16α)-Eburnamenin-14(15H)-one O-{2-[4-(2-methoxyphenyl)-1-piperaninyl]-ethyl}oxime or a non-toxic salt thereqf.

6 (3α, 16α)-Eburnamenin-14(15H)-one O-{2-hydroxy-3-[4-(2-methoxyphenyl)-1-piperadinyl]propyl}oxime or a non-toxic salt thereof.

7. A process for the preparation of an eburnamonine oxime represented by the formula

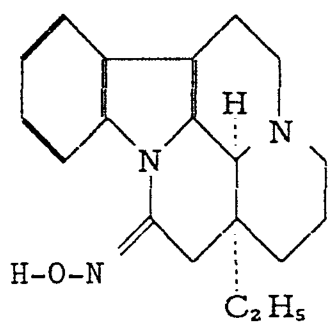

or a non-toxic salt thereof, which comprises reacting eburnamoninethione represented by the formula

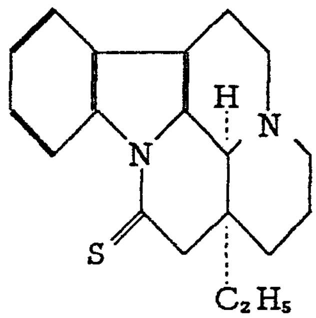

with hydroxylamine hydrochloride in an organic solvent and in the presence of an alkali metal carbonate, and if necessary converting the resulting free base into the non-toxic salt.

8. A process for the preparation of an eburnamonine oxime derivative represented by the formula

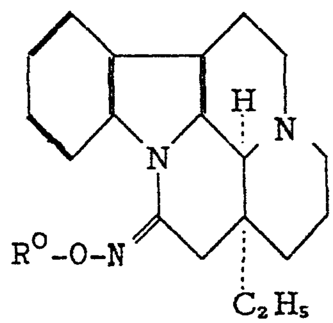

wherein $R^0$ is as defined for R in Claim 1, except that it is not hydrogen or the group (b),

or a non-toxic salt thereof, which comprises (i) reacting eburnameninthione represented by the formula

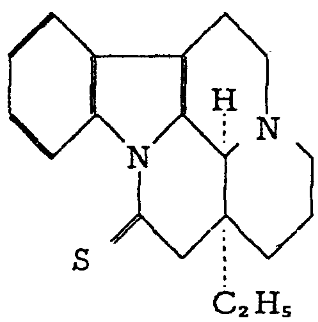

with hydroxylamine hydrochloride in an organic solvent and in the presence of an alkali metal carbonate, (ii) reacting in an anhydrous organic solvent the resulting eburnamonine oxime represented by the formula

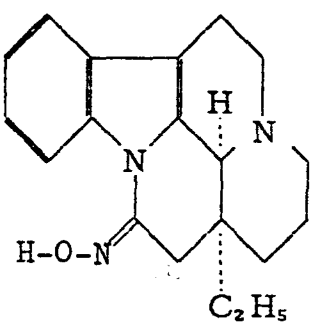

with an alkali metal hydride, and (iii) further reacting in an anhydrous organic solvent the resulting alkali metal salt of the eburnamonine oxime with a compound represented by the formula

$R^0X$

wherein $R^0$ has the meaning defined above and X is halogen and if necessary, converting the resulting free base to the non-toxic salt.

**9. A process for the preparation of** an eburnamonine oxime **derivative represented by the formula**

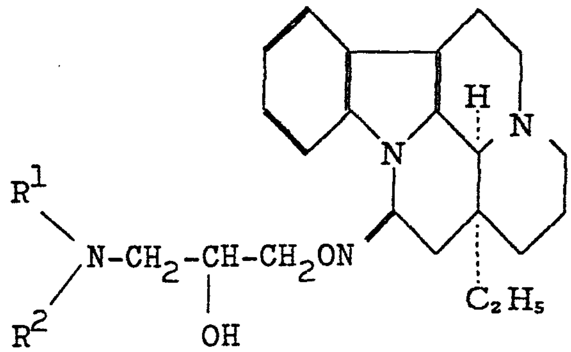

**werein $R^1$ and $R^2$ are** as defined in Claim 1, and non-toxic salts thereof, which comprises steps (i) and (ii) of the process of Claim 8,
and (iii) reacting in an anhydrous organic solvent **the resulting alkali metal salt of the eburnamonine oxime with a compound represented by the formula**

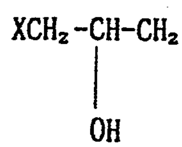

**wherein X is** a halogen
**and then with a compound represented by the formula**

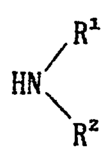

**wherein $R^1$ and $R^2$ have the meanings** defined in Claim 1
**and if necessary, converting the resulting free base into the non-toxic salt.**

**10. A cerebral function improving** pharmaceutical composition which comprises as an effective component an eburnamonine oxime drivative as claimed in any of Claims 1 to 6.